# EUROPEAN PATENT SPECIFICATION

(11) **EP 1 154 985 B1**
(45) Date of publication and mention of the grant of the patent: **14.04.2004**
(21) Application number: 00912451.2
(22) Date of filing: 04.02.2000
(51) Int. Cl.: C07C 231/02, C07C 233/18

(54) **PROCESS FOR THE INDUSTRIAL PRODUCTION OF BETA-HYDROXYALKYL AMIDES**
VERFAHREN ZUR INDUSTRIELLEN HERSTELLUNG VON BETA-HYDROXYALKYLAMIDEN
PROCEDE DE PRODUCTION INDUSTRIELLE D'AMIDES DE BETA-HYDROXYALKYLE

(30) Priority: 22.02.1999 IT MI990352
(43) Date of publication of application: 21.11.2001
(73) Proprietor: Sir Industriale S.p.A., 27100 Pavia (IT)
(72) Inventor: ROSSI, Pietro, Paolo, I-27026 Garlasco (IT); MARELLI, Gino, I-22031 Albavilla (IT); BALDINI, Alberto, I-27026 Garlasco (IT)
(74) Representative: Gervasi, Gemma, Dr.
(86) International application number: PCT/EP2000/000901
(87) International publication number: WO 2000/050384

(56) References cited:
- EP-A- 0 473 380

## Description

### Prior art

Processes are known for the preparation of β-hydroxyalkyl amides in which the first stage consists of the esterification of an organic acid with a monovalent alcohol.

In this reaction, it is necessary to use an excess of alcohol and to operate in the presence of acid catalysts.

These two aspects represent unfavourable characteristics both from the operating standpoint and from the economic standpoint, in that the excess of alcohol and the catalysts must be removed from the reaction mixture before the subsequent stage.

The second stage consists in the amidation reaction of the ester obtained in the first stage, using diethanolamine. The reaction may be performed in solvents or without solvents (dry) in the presence of catalysts as described in J. Coat Tech. 50(643) 49 - 55 (1978); US -A - 4,032,460; US - A - 4,076,917; US - A - 4,727,111, and EP 0473380 A1.

When operating in the absence of solvents, especially in industrial preparations where the quantities of the reagents may even exceed one tonne, there arise serious problems of disposal of the reaction heat, in that, in order to prevent the formation of by-products, it is necessary to maintain exactly the set reaction temperature.

As regards catalysts, which generally are strong bases, such as KOH or CH₃ONa, when operating in the absence of a solvent inevitably they remain englobed in the amide together with non-negligible quantities of non-reacted diethanolamine, with undesirable consequences in terms of application. In fact, β-hydroxyalkyl amides are used as cross-linking agents of polyester resins, and the presence of KOH or CH₃ONa may cause hydrolysis of the polyester.

This problem is particularly felt in the case of polyester-resin powder paints for outdoor use, for example for use on aluminium section, in that they are exposed to atmospheric agents.

In addition, besides contributing to the hydrolytic effects referred to above, the non-reacted diethanolamine that remains englobed in the β-hydroxyalkyl amide, during the phase of application of the β-hydroxyalkyl amide itself as cross-linking agent of powder polyester resins carried out in ovens at a relatively high temperature (150-200°C), gives rise to phenomena of yellowing that are particularly harmful and unacceptable in the case of white or transparent paints.

The negative effect of the presence of free diethanolamine is shown, for example, in Patent EP 0473380A1, in which diethanolamine is reduced to 1.6 -2% as compared to the prior art, in which it was higher than 2%.

### Summary of the invention

Now a process has been found for the industrial production of β-hydroxyalkyl amides which makes it possible to overcome the drawbacks of the prior art.

The said process comprises:
a) esterification reaction of a dicarboxylic organic acid with a glycol to obtain the corresponding polyester;
b) amidation reaction of the polyester obtained in stage a) with a β-hydroxyalkyl amine to obtain the corresponding β-hydroxyalkyl amide;
c) crystallization of the β-hydroxyalkyl amide thus obtained.

The reaction of stage b) is carried out on the reaction mixture of stage a) without separation of the polyester.

The characteristics and advantages of the process according to the present invention will be further highlighted in the course of the ensuing detailed description.

### Brief description of the figures

Figure 1 represents the plant for the process of the invention, in which:
   R 01 is the reactor in which the stages for preparation of the product are carried out;
   F 04 is a filtering apparatus for the separation of the product from the solvents used in the preparation.
Figure 2 represents the infrared spectrum of the product obtained in Example 1.
Figure 3 represents the infrared spectrum of the product obtained in Example 3.

### Detailed description of the invention

The present invention refers to a process for the industrial production of β-hydroxyalkyl amides.

The said process comprises:
- a first stage in which a dicarboxylic organic acid is made to react with a glycol to obtain the corresponding polyester;
- a second stage in which the said polyester is made to react with a β-hydroxyalkyl amine to obtain the corresponding β-hydroxyalkyl amide;
- a third stage in which the reaction mixture obtained in the second staqe containing the crystallized product is filtered, and the product is washed and dried.

The reaction of the second stage is carried out on the reaction mixture of the first stage and in the same reactor without separation of the polyester.

The said dicarboxylic acid is preferably adipic acid, but may also be azelaic acid, dodecandioic acid, suberic acid or sebacic acid.

The said glycol is preferably ethylene glycol, but may also be propylene glycol neopentyl glycol.

The reaction of the first stage is carried out with a molar ratio between the glycol and the acid approximately equal to the stoichiometric ratio, so as to form a polyester between the dicarboxylic acid and the glycol, at a temperature of between 190 and 230°C, in the first 10 hours without any solvent, and in the following 4 hours in the presence of small amounts of toluene, which serves as a means to remove the reaction water, possibly also in vacuum conditions.

The product obtained from the first stage is a polyester consisting of at least 3 molecules of glycol and 2 molecules of dicarboxylic acid, with the condition that the number of molecules of glycol is higher by one unit than the number of molecules of dicarboxylic acid, so that the polyester may be terminated prevalently with OH.

Preferably, the said polyester is made up of 11 molecules of glycol and 10 molecules of dicarboxylic acid.

The reaction of the second stage is carried out with a molar ratio between the dicarboxylic acid contained in the said polyester and the β-hydroxyalkyl amine of between 0.4:1 and 0.6:1 at a temperature of between 15 and 110°C in the presence of toluene, possibly in a mixture with 2-methyl-1-propanol or with a low-boiling alcohol, such as methanol. The reaction time may range from 4 to 15 hours.

In this reaction, a catalyst is used consisting of hydrazine, or else sodium methoxide or another alcoholate of an alkaline metal or a strong base, such as KOH or NaNH₂.

From this reaction, the β-hydroxyalkyl amide is obtained by amide scission of the ester bonds of the polyester chain.

Crystallization of the product is completed by means of cooling to 5°C.

The mixture obtained by means of the crystallization is filtered under pressure, and the solid product is washed with toluene, possibly in a mixture with 2-methyl-1-propanol or with a low-boiling ketone, such as acetone or methyl ethyl diketone or methyl isobutyl ketone.

The washed product is then vacuum dried at 40-60°C.

A product is obtained having a high degree of purity and a total alkaline content of less than 0.5%.

This product can be used as cross-linking agent in the preparation of polyester resins.

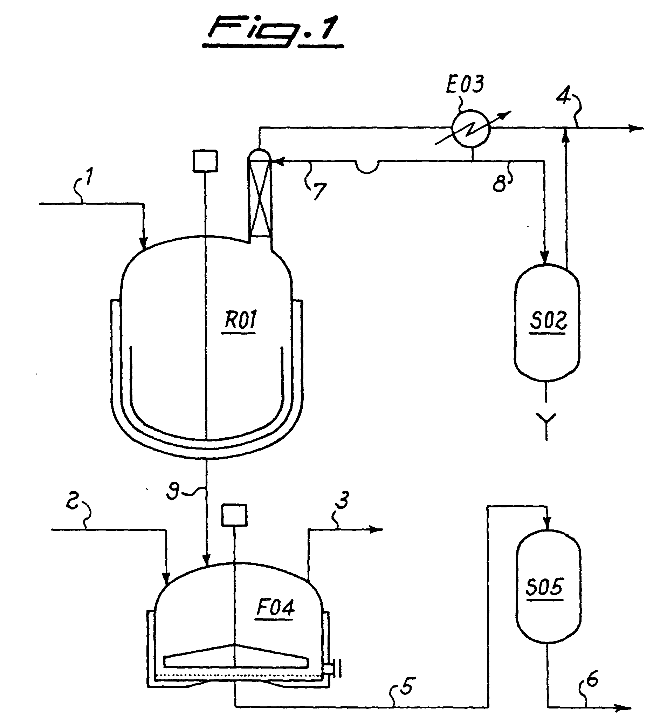

With reference to Figure 1, the first stage and second stage of the process are carried out in the reactor R 01 equipped with an agitator and a heating or cooling system.

No purification of the polyester is required in the passage from stage 1 to stage 2.

Filtration, washing and drying of the product are carried out in the apparatus F 04 equipped with filtering diaphragm, agitator, heating system, a line for feeding in pressurized nitrogen (2), and a vacuum line (3).

Among the numerous advantages of the process according to the present invention as compared to the prior art, the following must be particularly emphasized:
- in the first stage it is not necessary to purify the polyester and there is no alcohol to be separated;
- in the second stage, the reaction can be easily controlled and thermo-regulated at the desired value, normally ranging between 15 and 110°C, and more preferably between 30 and 95°C, by means of ordinary systems of indirect cooling of the reactor, or also in vacuum conditions, and by means of the consequent precipitation of the solvent or of the mixture of solvents;
- the product obtained shows exceptional purity, with a content of free β-hydroxyalkyl amine lower than 1.5%.

### Example 1 (diethanolamide of adipic acid)

The processes described in the present example and in the following examples were carried out using the system illustrated in Figure 1.

### Stage a): Esterification reaction

In the reactor R 01 having a volume of 14 m³ and equipped with a stirring system and a heating system, are introduced, using line (1 ), 2,200 kg of adipic acid and 1,000 kg of glycol.

The mixture is heated up to 200°C in 6 hours, and the water produced by the esterification reaction is collected in the tank S 02.

After 8 hours, when the reaction slows down, the reaction mixture is diluted with 500 litres of toluene, which is added slowly so as to maintain the temperature of the mixture at 200°C.

After 6 hours, during which a mixture consisting of reaction water and toluene is distilled, the reaction is complete (residual acidity of the ester < 5 mg of KOH/g).

### Stage b): Amidation reaction

The mixture obtained in stage a) is cooled down to 100°C in the reactor R 01 and is then diluted with 4,200 kg of toluene and 4,200 kg of 2-methyl-1-propanol, and 3,150 kg of diethanolamine and 40 kg of hydrazine hydrate are added to it.

The mixture is kept at 100-105°C at a pressure of 680-700 mmHg (90.44 - 93.21 KPa) for 18 hours, and then the vacuum level is increased via the line (4).

The mixture of solvents that evaporates is condensed and flows back into the reactor R 01 via the line (7).

Vacuum evaporation of the mixture of solvents causes cooling of the reaction mixture and crystallization of the amide produced. When the residual pressure reaches 5 mmHg (667 Pa), the temperature of the reaction mixture is approximately 5°C.

### Stage c): Filtration, washing and drying

The reactor R 01 is fed with nitrogen up to a pressure of 760 mmHg (101.30 KPa). The reaction mixture is transferred via the line (9) into the filtering apparatus F 04 equipped with stirring system and heating system.

The mixture is filtered under a pressure of 3 atmospheres of nitrogen, and the filtered solvent is collected in the tank S 05.

The solid product is washed twice in F 04 with 500 kg of a 1:1 mixture of toluene and 2-methyl-1-propanol.

At the end of washing, the product is vacuum dried at 40-50°C, the vacuum being obtained via the line (3), and kept under stirring for 5 hours.

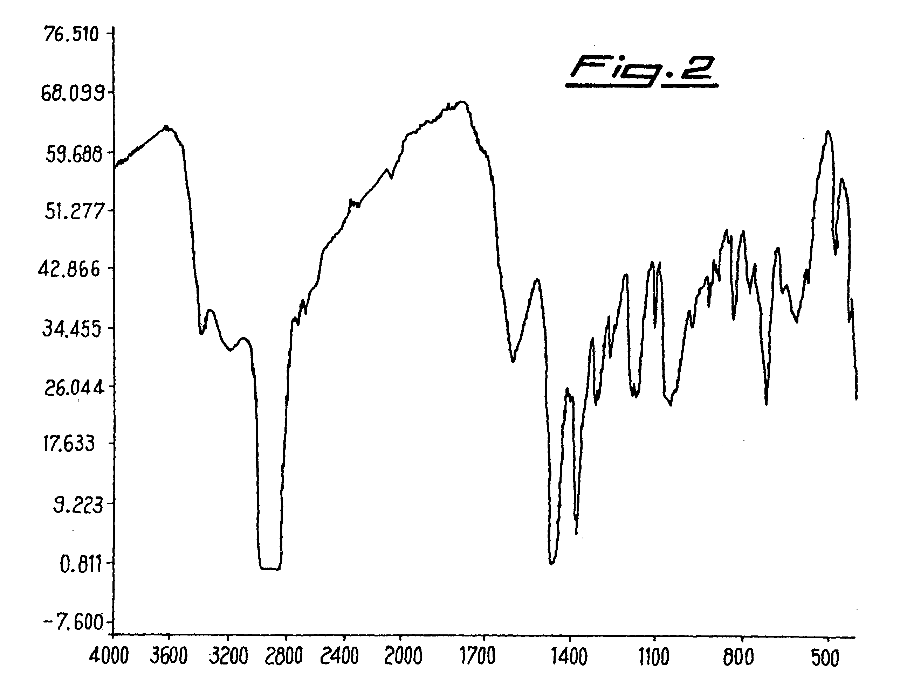

In this way, 3,800 kg of diethanolamide of adipic acid are obtained in the crystalline form with a content of free diethanolamine lower than 0.5% and with a melting temperature of 121-122 °C. The infrared spectrum (in NUJOL) of the product obtained is shown in Figure 2. It may be noted that the band of absorption of the ester is absent.

### Example 2 (diethanolamide of adipic acid)

### Stage a): Esterification reaction

In the reactor are introduced 1,168.8 kg of adipic acid (8 kmol) and 536 kg of ethylene glycol (8.64 kmol), and polyesterification is carried out as described in Example 1, with the variant that instead of using toluene to remove the reaction water, vacuum is applied at 200°C for 8 hours.

### Stage b): Amidation reaction

The mixture obtained in stage a) is cooled to 100°C, and 3,200 kg of toluene, 1,680 kg of diethanolamine and 480 kg of sodium methylate as a 5% solution in methanol are added. The temperature is kept at 50-55°C. After 30 minutes, there takes place the separation of a gelatinous mass dispersed in the toluene. To this, 800 kg of isobutyl alcohol are added, and within 12 hours a white crystalline suspension is obtained. The product is cooled down by circulating refrigerated water in the reactor cooling system until the inside temperature reaches 10°C.

### Stage c): Filtration, washing and drying

The reaction mixture obtained in stage b) is filtered; and the crystals are washed twice with a solution consisting of 300 kg of toluene and 75 kg of isobutyl alcohol, and then are vacuum dried. In this way, 2,300 kg of crystals of diethanolamide of adipic acid are obtained having a melting temperature of 118-120°C and containing 0.9% free diethanolamine.

### Example 3 (diethanolamide of suberic acid)

### Stage a): Esterification reaction

The following are introduced into the reactor R 01:
1,045.2 kg of suberic acid (1,6-hexanedicarboxylic acid), corresponding to 6 kmol, and 409.2 kg of ethylene glycol, corresponding to 6.6 kmol. The mixture is heated up to 212-215°C so as to distil the reaction water in approximately 6 hours. At this point, vacuum is applied, and the mixture is kept under vacuum conditions at this temperature for a further 8 hours. The vacuum is removed with nitrogen, and the free acidity still present in the reaction mass is titred. This corresponds to 2 mg of KOH per gram of polyester.

### Stage b): Amidation reaction

The polyester obtained in stage a) is cooled down to 80°C, and then 1,260 kg of diethanolamine, corresponding to approximately 12 kmol, and 600 kg of methanol are added.

At this point, the temperature is 48°C.

Next, 144 kg of a 30% solution of sodium methylate in methanol are added, the temperature is increased and subsequently the methanol is made to distil until the temperature reaches 90°C. This temperature is maintained by refluxing the methanol to the reactor R 01 via the line 7 for 4 hours. The mixture is then cooled to 50°C, and 2,400 kg of methyl ethyl ketone are added. The product is further cooled to 10°C in 4 hours, and this temperature is maintained for a further 4 hours.

### Stage c): Filtration, washing and drying

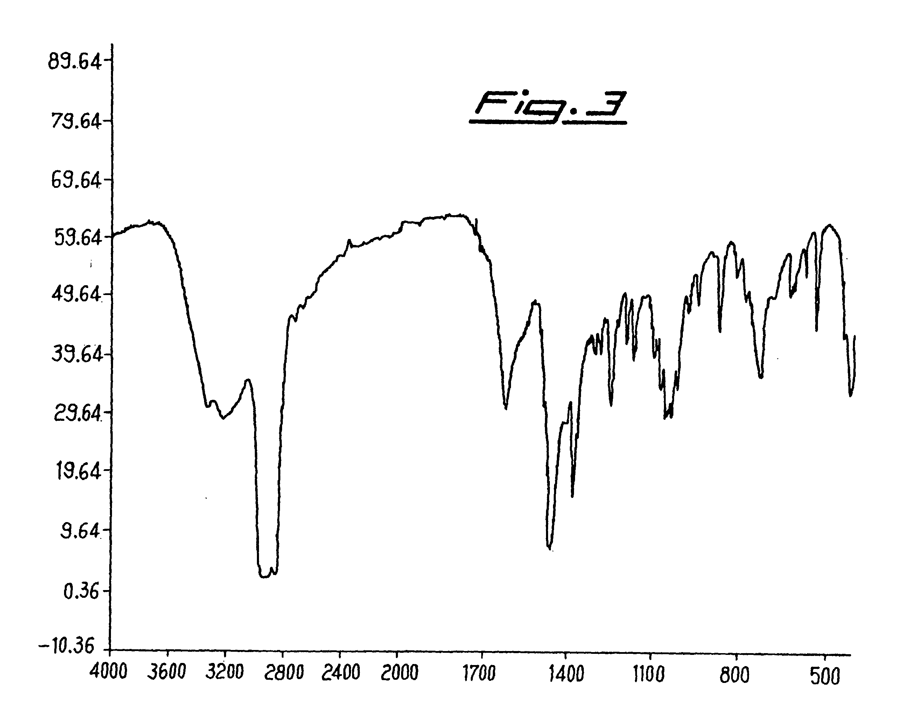

The suspension of crystals of the diethanolamide of suberic acid is transferred into the filter F 04. The crystallization solvent is removed by applying a pressure of up to 2 nitrogen atmospheres on the filter, and the panel is washed twice with 1,200 kg at a time of methyl ethyl diketone. The crystals are then vacuum dried to obtain finally 1,850 kg of diethanolamide of suberic acid with a melting point of 84-86°C and containing 0.8% of free diethanolamine. The infrared spectrum (in NUJOL) of the product obtained is shown in Figure 3. It may be noted that the band of absorption of the ester is absent.

## Claims

1. Process for the industrial production of β-hydroxyalkyl amides **characterized in that**:
a) dicarboxylic organic acid is made to react with a glycol to obtain the corresponding polyester;
b) the polyester obtained in stage a) is made to react with a β-hydroxyalkyl amine to obtain the corresponding β-hydroxyalkyl amide, using a basic catalyst;
c) the reaction mixture obtained in stage b) undergoes crystallization.

2. Process according to Claim 1, **characterized in that** the said polyester consists of at least 3 molecules of glycol and 2 molecules of dicarboxylic acid.

3. Process according to Claim 1, **characterized in that** the said polyester consists of 11 molecules of glycol and 10 molecules of dicarboxylic acid.

4. Process according to Claim 1, **characterized in that** the reaction of stage b) is carried out on the reaction mixture of stage a) and in the same reactor without separation of the polyester.

5. Process according to Claim 1, **characterized in that** the said dicarboxylic acid is chosen from the group comprising adipic acid, suberic acid, sebacic acid, azelaic acid, and dodecandioic acid.

6. Process according to Claim 1, **characterized in that** the said glycol is chosen from the group comprising ethylene glycol, propylene glycol and neopentyl glycol.

7. Process according to Claim 1, **characterized in that** the said β-hydroxyalkyl amine is diethanolamine.

8. Process according to Claim 1, **characterized in that** the said basic catalyst is chosen from among hydrazine, an alcoholate of an alkaline metal, KOH, and NaNH₂.

9. Process according to Claim 1, **characterized in that** the reaction of stage a) is carried out at a temperature of between 190 and 230°C, initially without any solvent, and subsequently in the presence of toluene or of vacuum.

10. Process according to Claim 1, **characterized in that** the reaction of stage b) is carried out with a molar ratio of from 0.4:1 to 0.6:1, preferably from 0.45:1 to 0.55:1, between the acid contained in the said polyester and the β-hydroxyalkyl amine, at a temperature of between 15 and 110°C.

11. Process according to Claim 1, **characterized in that** the said crystallization of stage c) is carried out by means of cooling down to 5°C.

## Patentansprüche

1. Verfahren zur Herstellung von Beta-Hydroxyalkylamiden, **dadurch gekennzeichnet, dass**:
a) eine Dicarbonsäure mit einem Glykol zur Reaktion gebracht wird, um den entsprechenden Polyester zu erhalten;
b) der in der Stufe a) erhaltene Polyester mit einem Beta-Hydroxyalkylamin zur Reaktion gebracht wird, um das entsprechende Beta-Hydroxyalkylamid zu erhalten, wobei ein basischer Katalysator verwendet wird;
c) das in der Stufe b) erhaltene Reaktionsgemisch zur Kristallisation gebracht wird.

2. Verfahren gemäß Anspruch 1, **dadurch gekennzeichnet, dass** der genannte Polyester aus wenigstens 3 Glykolmolekülen und 2 Dicarbonsäuremolekülen besteht.

3. Verfahren gemäß Anspruch 1, **dadurch gekennzeichnet, dass** der genannte Polyester aus 11 Glykolmolekülen und 10 Dicarbonsäuremolekülen besteht.

4. Verfahren gemäß Anspruch 1, **dadurch gekennzeichnet, dass** die Reaktion von Stufe b) mit dem Reaktionsgemisch aus Stufe a) und in demselben Reaktor ohne Abtrennung des Polyesters erfolgt.

5. Verfahren gemäß Anspruch 1, **dadurch gekennzeichnet, dass** die genannte Dicarbonsäure aus der folgenden Gruppe ausgewählt ist: Adipinsäure, Korksäure, Sebacinsäure, Azelainsäure und Dodecandisäure.

6. Verfahren gemäß Anspruch 1, **dadurch gekennzeichnet, dass** das genannte Glykol aus der folgenden Gruppe ausgewählt ist: Ethylenglykol, Propylenglykol und Neopentylglykol.

7. Verfahren gemäß Anspruch 1, **dadurch gekennzeichnet, dass** das genannte Beta-Hydroxyalkylamin Diethanolamin ist.

8. Verfahren gemäß Anspruch 1, **dadurch gekennzeichnet, dass** der genannte basische Katalysator aus der folgenden Gruppe ausgewählt ist: Hydrazin, ein Alkalialkoholat, KOH und NaNH₂.

9. Verfahren gemäß Anspruch 1, **dadurch gekennzeichnet, dass** die Reaktion der Stufe
a) bei einer Temperatur zwischen 190 und 230 °C erfolgt, zu Beginn ohne jegliches Lösungsmittel und darauf in der Gegenwart von Toluol oder unter Vakuum.

10. Verfahren gemäß Anspruch 1, **dadurch gekennzeichnet, dass** das Molverhältnis zwischen der in dem genannten Polyester enthaltenen Säure und dem Beta-Hydroxyalkylamin in der Reaktion der Stufe b) im Bereich von 0,4:1 bis 0,6:1, vorzugsweise von 0,45:1 bis 0,55:1 liegt und die Reaktion bei einer Temperatur zwischen 15 und 110 °C erfolgt.

11. Verfahren gemäß Anspruch 1, **dadurch gekennzeichnet, dass** die Kristallisation der Stufe c) durch Abkühlen auf 5 °C erfolgt.

## Revendications

1. Procédé pour la production industrielle de β-hydroxyalkyl amides **caractérisé en ce que** :
a) un acide dicarboxylique organique est forcé à réagir avec un glycol pour obtenir le polyester correspondant ;
b) le polyester obtenu au stade a) est forcé à réagir avec une β-hydroxyalkyl amine pour obtenir le β-hydroxyalkyl amide correspondant en utilisant un catalyseur basique ;
c) le mélange réactionnel obtenu au stade b) subit une cristallisation.

2. Procédé selon la revendication 1, **caractérisé en ce que** ledit polyester consiste en au moins 3 molécules de glycol et 2 molécules d'acide dicarboxylique.

3. Procédé selon la revendication 1, **caractérisé en ce que** ledit polyester consiste en 11 molécules de glycol et 10 molécules d'acide dicarboxylique.

4. Procédé selon la revendication 1, **caractérisé en ce que** la réaction du stade b) est effectuée sur le mélange réactionnel du stade a) et dans le même réacteur sans séparation du polyester.

5. Procédé selon la revendication 1, **caractérisé en ce que** ledit acide dicarboxylique est choisi dans le groupe comprenant de l'acide adipique, de l'acide subérique, de l'acide sébacique, de l'acide azélaïque, et de l'acide dodécanedioïque.

6. Procédé selon la revendication 1, **caractérisé en ce que** ledit glycol est choisi dans le groupe comprenant éthylène glycol, propylène glycol et néopentyl glycol.

7. Procédé selon la revendication 1, **caractérisé en ce que** ladite β-hydroxyalkyl amine est la diéthanolamine.

8. Procédé selon la revendication 1, **caractérisé en ce que** ledit catalyseur basique est choisi parmi hydrazine, un alcoolate d'un métal alcalin, KOH, et NaNH₂.

9. Procédé selon la revendication 1, **caractérisé en ce que** la réaction du stade a) est effectuée à une température comprise entre 90 et 230°C, initialement sans aucun solvant, et subséquemment en présence de toluène ou de vide.

10. Procédé selon la revendication 1, **caractérisé en ce que** la réaction du stade b) est effectuée avec un rapport molaire de 0,4:1 à 0,6:1, de préférence de 0.45:1 à 0,55:1, entre l'acide contenu dans ledit polyester et la β -hydroxyalkyl amine, à une température entre 15 et 110°C.

11. Procédé selon la revendication 1, **caractérisé en ce que** ladite cristallisation du stade c) est effectuée au moyen d'un refroidissement à 5°C.
